# EUROPEAN PATENT SPECIFICATION

(11) **EP 3 192 492 B1**
(45) Date of publication and mention of the grant of the patent: **13.11.2019**
(21) Application number: 15840955.7
(22) Date of filing: 09.09.2015
(51) Int. Cl.: A61K 8/31, A61K 8/37, A61K 8/891, A61K 8/92, A61Q 1/04

(54) **SOLID LIP COSMETIC**
FESTES LIPPENKOSMETIKUM
PRODUIT COSMÉTIQUE SOLIDE POUR LES LÈVRES

(30) Priority: 09.09.2014 JP 2014183612
(43) Date of publication of application: 19.07.2017
(73) Proprietor: Shiseido Company, Ltd., Tokyo 104-0061 (JP)
(72) Inventor: NAKANO, Yusuke, YOKOHAMA-SHI Kanagawa 224-8558 (JP); TOMITA, Noriko, YOKOHAMA-SHI Kanagawa 224-8558 (JP); SUZUKI, Sumire, YOKOHAMA-SHI Kanagawa 224-8558 (JP)
(74) Representative: Ter Meer Steinmeister & Partner
(86) International application number: PCT/JP2015/075575
(87) International publication number: WO 2016/039369

(56) References cited:
- EP-A1- 2 716 277
- WO-A1-2012/165130
- JP-A- H1 036 223
- JP-A- 2011 140 481
- US-A1- 2007 134 181
- US-A1- 2012 237 467

## Description

### RELATED APPLICATIONS

This application claims the priority of Japanese Patent Application No. 2014-183612 filed on September 9, 2014, which are incorporated herein by reference.

### FIELD OF THE INVENTION

The present invention relates to a solid lip cosmetic, and in particular, relates to a lip cosmetic having excellent secondary adhesion protective effect immediately after being applied and being excellent in gloss (luster) durability and stability.

### BACKGROUND OF THE INVENTION

Conventional lip cosmetics have faced the problem of secondary adhesion, namely a lipstick content is transferred onto a site contacted by a lip (for example, a cup (mug)) after the lipstick is applied to the lip. By contrast, lip cosmetics having so-called secondary adhesion protective effect that causes little secondary adhesion have been developed.

For example, in Patent Literatures 1 to 3, by using lip-adhering hydrogenated polyisobutene and organic silicone oil that has low compatibility with the hydrogenated polyisobutene at room temperature, solid lip cosmetics, which are homogeneous in the product but the hydrogenated polyisobutene and the organic silicone oil separate by contact during application, have been disclosed.

In the lip cosmetics having the secondary adhesion protective effect, hydrogenated polyisobutene adheres to the skin (lip) during use, and organic silicone oil forms a film on the hydrogenated polyisobutene layer. This organic silicone oil is colorless and transparent; therefore, a smudge is not noticeable even when it is transferred to a cup etc.; thus the secondary adhesion protective effect can be exhibited.

The blending of coloring material is normally necessary for a solid lip cosmetic to function as a makeup cosmetic. The coloring material is directed more to hydrogenated polyisobutene than to organic silicone oil; therefore, the percentage of hydrogenated polyisobutene needs to be large to blend a certain amount of coloring material. Furthermore, hydrogenated polyisobutene itself is colorless and transparent, and it is an oil having various viscosities depending upon the degree of polymerization. Thus, when a large amount of high-viscosity hydrogenated polyisobutene is blended, the hydrogenated polyisobutene layer of lipstick tends to likely remain more on the lip, and the long-lasting property is improved.

However, when the percentage of hydrogenated polyisobutene is increased, the separation of organic silicone oil becomes difficult during application. Not only the secondary adhesion protective effect is lowered, but also it tended to become difficult to mold a stick with a shape. Therefore, in the past, the blending quantity of hydrogenated polyisobutene has been virtually limited to 30 % by mass or lower.

Patent Literature 4 relates to a solid cosmetic for lips characterized by comprising (a) 5 to 30 mass % of hydrogenated polyisobutene, (b) 30 to 70 mass % of one or more kinds of methyl phenyl silicones that separate from (a) when mixed therewith at 25 °C, (c) 0.5 to 15 mass % of oil that separates from both (a) and (b) when mixed therewith at 25 °C, and (d) 4 to 12 mass % of wax.

### [PRIOR ART DOCUMENTS]

### [PATENT LITERATURES]

[Patent Literature 1] Japanese Patent No. 4757950
[Patent Literature 2] Japanese Patent No. 5280490
[Patent Literature 3] Japanese Unexamined Patent Publication No. 2012-82188
[Patent Literature 4] EP 2 716 277 A1

### [SUMMARY OF THE INVENTION]

### [PROBLEM TO BE SOLVED BY THE INVENTION]

The present invention was made in view of the above-described problem of the conventional art, and an object is to provide a solid lip cosmetic that has an excellent secondary adhesion protective effect and can provide the increased freedom in the blending quantity of hydrogenated polyisobutene.

### [MEANS TO SOLVE THE PROBLEM]

The present inventors have diligently studied to solve the above-described problem; as a result, the present inventors have found that the freedom in the selection of the blending quantity of hydrogenated polyisobutene can be increased without losing the secondary adhesion protective effect by choosing a compatibility modifier, which is blended to improve the dispersion of hydrogenated polyisobutene and organic silicone oil during production (during heating at a high temperature), thus the present invention is completed.

In order to achieve the above-described object, the solid lip cosmetic of the present invention comprises the following components (a) to (d):
(a) 25 to 45 % by mass of hydrogenated polyisobutene,
(b) 20 to 70 % by mass of one or more methyl phenyl silicones that separate at 25 °C when mixed with (a),
(c) 1 to 20 % by mass of neopentyl glycol dicaprate, as the compatibility modifier, which modifies the compatibility of component (a) and component (b), and
(d) 3 to 12 % by mass of wax.

It is preferable that the solid lip cosmetic comprise (e) a coloring material is contained.

It is preferable that the solid lip cosmetic comprise component (e) is dispersed in component (a). It is preferable that the solid lip cosmetic comprise methyl phenyl silicone of component (b) include one or more selected from the group consisting of trimethyl pentaphenyl trisiloxane, diphenyl dimethicone, diphenylsiloxy phenyl trimethicone, and phenyl trimethicone.

It is preferable that the solid lip cosmetic comprise one or more selected from the group consisting of olefin oligomer, glyceryl tri-2-ethylhexanoate, sorbitan sesquiisostearate, propylene glycol monostearate, cetyl PEG/PPG-10/1 dimethicone, diglyceryl diisostearate, glyceryl diisostearate, pentaerythrityl tetraethylhexanoate, squalane, liquid paraffin, trimethylolpropane triisostearate, trimethylolpropane triethylhexanoate, diisostearyl malate, and cetyl ethylhexanoate are further contained as the (f) compatibility modifier aid.

### [EFFECT OF THE INVENTION]

In the solid lip cosmetic of the present invention, specific amounts of (a) hydrogenated polyisobutene, (b) one or more methyl phenyl silicones that separate at 25 °C when mixed with (a), (c) neopentyl glycol dicaprate, and (d) wax are blended, Thus, a solid lip cosmetic excellent in the secondary adhesion resistance effect, long-lasting property, and moldability can be obtained while expanding the range of choice for the blending quantity of hydrogenated polyisobutene.

### [BRIEF DESCRIPTION OF DRAWINGS]

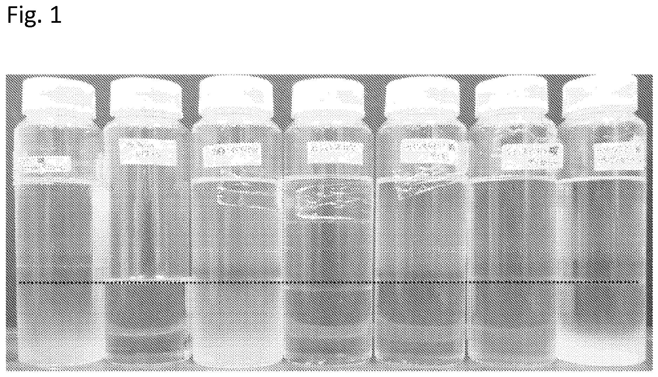

Fig. 1 is an explanatory drawing of the affinity of various compatibility modifiers or aids to hydrogenated polyisobutene or methyl phenyl silicone.

### [MODES FOR CARRYING OUT THE INVENTION]

The solid lip cosmetic of the present invention comprises (a) hydrogenated polyisobutene, (b) one or more methyl phenyl silicones that separate at 25 °C when mixed with (a), (c) neopentyl glycol dicaprate, and (d) wax.

Hereinafter, the respective components will be explained in detail.

### (a) Hydrogenated polyisobutene

Component (a) is an oil that does not dissolve in component (b). Component (a) has a higher affinity to the lip than component (b), and it likely adheres on the lip.

It is preferable that the average molecular weight of the hydrogenated polyisobutene is 1000 to 2650.

In particular, the effect due to the selection of the below-described neopentyl glycol dicaprate is prominently exhibited when the blending quantity of hydrogenated polyisobutene is 30% by mass or higher. That is, when the blending quantity of component (a) is 30% by mass or higher, the long-lasting property can be further improved while the secondary adhesion protective effect is maintained.

When the better secondary adhesion protective effect and long-lasting property are desired, it is preferable that the blending quantity of component (a) is 45% by mass or lower.

If the amount of hydrogenated polyisobutene in the total cosmetic is less than 25% by mass, the coloring material in the cosmetic cannot sufficiently be directed to the hydrogenated polyisobutene layer, and the blending quantity of the coloring material is limited. If the amount exceeds 50% by mass, moldability becomes poor, and a problem may be generated in the feeling in use such as a poor long-lasting property and the generation of stickiness; in addition, the amount of methyl phenyl silicone becomes low correspondingly, so that it can be difficult that the secondary adhesion protective effect becomes clear.

### (b) Methyl phenyl silicone

(b) methyl phenyl silicone used in the present invention separates when mixed with (a) hydrogenated polyisobutene at 25 °C.

The (b) methyl phenyl silicone blended in the solid lip cosmetic of the present invention can be one kind or a mixture of two or more kinds.

Here, the presence or absence of "separation" was measured under the following conditions.

### (Measurement condition)

(a) and (b) were used in the ratio ((a):(b) = 1:1 (mass ratio)) and heated to 90 °C. After the mixture was mixed under stirring, it was allowed to stand at 25 °C. When the boundary was uniformly separated into two layers, it was denoted "separated". When it was a translucent state or a transparently miscible state without a boundary, it was denoted "not separated".

When two or more methyl phenyl silicones are used as component (b), the presence or absence of separation varies depending on the blending ratio thereof. Therefore, it is necessary to observe the presence or absence of separation according to a blending ratio of the component (b).

Examples of methyl phenyl silicones include trimethyl pentaphenyl trisiloxane, diphenyl dimethicone, diphenylsiloxy phenyl trimethicone, and phenyl trimethicone.

methyl pentaphenyl trisiloxane is preferable as (b) methyl phenyl silicone blended in the solid lip cosmetic of the present invention.

Examples of trimethyl pentaphenyl trisiloxanes as a commercial product include methyl phenyl silicone PH-1555 (180 mm2/s (25 °C), manufactured by Dow Corning Toray Co., Ltd.) and methyl phenyl silicone FZ3156 (165 mm2/s (25 °C), manufactured by Dow Corning Toray Co., Ltd.)

Furthermore, examples of diphenyl dimethicone as a commercially available product include silicone KF54 (400 mm2/s (25 °C), manufactured by Shin-Etsu Chemical Co., Ltd.), and silicone KF50-300CS (270 to 330 mm2/s (25 °C), manufactured by Shin-Etsu Chemical Co., Ltd.).

Examples of diphenylsiloxy phenyl trimethicones as a commercially available product include silicone KF56 (14 mm2/s (25 °C), manufactured by Shin-Etsu Chemical Co., Ltd.).

Examples of phenyl trimethicone as a commercial product include silicone SH556 (22 mm2/s (25 °C), manufactured by Dow Corning Toray Co., Ltd.).

It is necessary that the blending quantity of (b) hydrogenated polyisobutene is 20 to 70% by mass relative to the total amount of the cosmetic.

Furthermore, it is preferable that the blending quantity is 30% by mass or higher. In addition, it is preferable that the blending quantity is 60% by mass or lower. If the blending quantity of component (b) is less than 20% by mass, the separation during application becomes difficult, and the secondary adhesion protective effect is not manifested. If it exceeds 70% by mass, the blending quantity of other components decreases, and the blending of coloring material becomes difficult.

### (c) Neopentyl glycol dicaprate as the compatibility modifier

The compatibility modifier is an oil that is compatible with component (a) and component (b) at 90 °C, separates at 25 °C when mixed with component (a), and is compatible with or disperses when mixed with component (b). As the preferable compatibility modifier of the present invention, neopentyl glycol dicaprate can be listed.

Here, the presence or absence of "separation" was measured under the following conditions.

### (Measurement condition)

(a), (b) and (c) were used in the ratio ((a): (b): (c) = 3:4:1 (mass ratio)) and heated to 90 °C. After the mixture was mixed under stirring, it was allowed to stand at 25 °C. When the boundary was uniformly separated into two layers, it was denoted "separated". When it was a translucent state or a transparently miscible state without a boundary, it was denoted "not separated".

It is necessary that the blending quantity of (c) hydrogenated polyisobutene is 1 to 20% by mass relative to the total amount of the cosmetic.

Furthermore, it is preferable that the blending quantity is 2% by mass or higher.

Furthermore, it is especially preferable that the blending quantity is 5% by mass or higher.

In addition, it is preferable that the blending quantity is 15% by mass or lower.

If the blending quantity of component (c) is less than 1% by mass, (a) and (b) are not compatible at 90 °C; as a result, molding as a lipstick cannot be achieved. If the blending quantity exceeds 20% by mass, the secondary adhesion protective effect is not manifested.

By using the above-described components (a) to (c), when the solid lip cosmetic of the present invention is applied on the lip, component (a) and components (b) and (c) immediately separate upon the contact of the cosmetic and the lip, component (a) adheres on the lip, and components (b) and (c) with low viscosity separate to the surface layer; as a result, the secondary adhesion protective effect is manifested. When a contact with an object such as a cup takes place in the state that such a cosmetic is applied on the skin (lip), only the transparent components (b) and (c) in the cosmetic adhere to the object. In addition, because component (b) is present in a large amount, component (b) again separates to the surface layer upon the contact of the object and the lip. Accordingly, the solid lip cosmetic of the present invention can manifest the secondary adhesion protective effect for a long time.

### (d) Wax

(d) wax blended in the solid lip cosmetic of the present invention is not limited in particular as long as it can be normally blended for cosmetics.

It is preferable that the wax used in the present invention is compatible with the methyl phenyl silicone.

Examples of the waxes used in the present invention include carnauba wax, candelilla wax, polyethylene wax, beeswax, ceresin, microcrystalline wax, solid paraffin, and Japan wax.

The blending quantity of component (d) is 3 to 12% by mass relative to the total amount of the cosmetic and preferably 4 to 12% by mass. If the blending quantity of component (d) is less than 3% by mass, the solidification is difficult. If it exceeds 12% by mass, the cosmetic becomes heavily spreadable and the gloss thereof is lost.

In the solid lip cosmetic of the present invention, in addition to the above essential components (a) to (d), components normally used in lip cosmetics can be included as optional components.

In the solid lip cosmetic of the present invention, it is preferable to blend (e) coloring material.

Coloring materials can be powdery or lake-like (oil-containing state) so far as they are coloring materials normally used in lipsticks. They can be inorganic pigments, organic pigments, or pearlescent agents. Inorganic pigments, organic pigments, and pearlescent agents are all more wettable to the dispersed-phase oil component (component (a)) than to the continuous phase oil component (component (b)). Accordingly, the coloring material spontaneously moves into the dispersed-phase oil component. Therefore, the coloring material is held in (a) hydrogenated polyisobutene when the cosmetic is applied and it is present in the inner side of component (b) of surface layer; thus the secondary adhesion rarely takes place.

Thus, in the solid lip cosmetic of the present invention, it is preferable that component (e) is substantially dispersed in component (a). It is especially preferable that 80% by mass or more component (e) of the total amount of coloring material is dispersed in component (a).

The blending quantity of coloring material is preferably 1 to 15% by mass relative to the total amount of the cosmetic and especial preferably 5 to 10% by mass. If the blending quantity of coloring material is too small, the secondary adhesion protective effect may be hardly believed.

In the solid lip cosmetic of the present invention, it is preferable to blend, as the (f) compatibility modifier aid, one or more selected from the group consisting of olefin oligomer, glyceryl tri-2-ethylhexanoate, sorbitan sesquiisostearate, propylene glycol monostearate, cetyl PEG/PPG-10/1 dimethicone, diglyceryl diisostearate, glyceryl diisostearate, pentaerythrityl tetraethylhexanoate, squalane, liquid paraffin, trimethylolpropane triisostearate, trimethylolpropane triethylhexanoate, diisostearyl malate, and cetyl ethylhexanoate.

Component (f) has high compatibility with components (a) to (c) at a high temperature (90 °C), and a more stable solid lip cosmetic can easily be prepared by blending such an oil.

When component (f) is blended, the blending quantity is preferably 1 to 20 % by mass with respect to the total amount of the cosmetic, and especially preferably 1 to 15% by mass. If the blending quantity of component (f) is less than 1% by mass, the compatibility of components (a) and (b) is poor and separation may take place. If the blending quantity exceeds 20% by mass, component (b) does not separate during its application on the lip, and the secondary adhesion protective effect may not be manifested.

The addition of an adjuvant that exceeds the blending quantity of neopentyl glycol dicaprate, which is component (c), may lower the blending effect of neopentyl glycol dicaprate; therefore, it is preferably the equal amount or less.

In the solid lip cosmetic of the present invention, in addition to the above-described components, the components normally used in lip cosmetics (for example, oil other than the above-described oils, powder, polymer compound, moisturizer, perfume, antioxidant agent, preservative, and beauty component) can be blended so far as the effect of the present invention is not undermined.

It is preferable that the solid lip cosmetic of the present invention is constituted so that the separation does not take place throughout the entire production process and the state of one homogeneous phase is maintained.

The solid lip cosmetic of the present invention can be applied to lipsticks, lip glosses, lip bases, overcoats for lipsticks, lip creams, and the like. In particular, a solid lipstick is preferable.

### [EXAMPLES]

The present invention will be further described in the following examples. However, the invention is not limited by these examples. Unless otherwise specified, the blending quantity of each component will be expressed in % by mass.

Thus far, the present inventors have found that a solid lip cosmetic, wherein (a) hydrogenated polyisobutene and a large amount of (b) one or more methyl phenyl silicones that separate at 25 °C when mixed with (a) are solidified with wax, is excellent in the secondary adhesion protective effect.

That is, during its application on the lip, component (b) with low viscosity and low affinity to the lip separates to the surface layer, and component (a) is present in the inner layer (lip surface). The coloring material has a high affinity to component (a); therefore, the coloring material is taken in the inner layer. Therefore, when a lip cosmetic sticks on the cup, only component (b) where coloring material not blended adheres to the cup. Because component (b) is present in a large amount, component (b) exudes from the lip cosmetic upon the contact of the cup and the lip. Therefore, in the above-described system, an excellent secondary adhesion protective effect can be exhibited for a long time.

As described above, if the coloring material is localized in the methyl phenyl silicone layer, methyl phenyl silicone that adheres to a cup etc. becomes colored; thus it is necessary to localize the coloring material in the hydrogenated polyisobutene layer. In addition, it is preferable to blend a large amount of high-viscosity hydrogenated polyisobutene to make it easier for the cosmetic containing coloring material in the hydrogenated polyisobutene layer to remain on the lip.

Therefore, for the production of a solid lip cosmetic in which various coloring materials are blended, a large amount of hydrogenated polyisobutene needs to be blended.

In the above-described Patent Literatures1 to 3, compatibility modifiers such as sorbitan sesquiisostearate are compatible with (a) hydrogenated polyisobutene and (b) organic silicone oil at 90 °C, and they contribute to the compatibility of hydrogenated polyisobutene and organic silicone oil. However, the compatibility modifier of the above-described Patent Literatures 1 to 3 generally tend to have a higher affinity to hydrogenated polyisobutene than to methyl phenyl silicone at ordinary temperature (25 °C).

Therefore, it is considered that hydrogenated polyisobutene was diluted by the compatibility modifier in the actual product; as a result, the secondary adhesion protective effect and moldability were lowered.

Thus, the present inventors selects neopentyl glycol dicaprate, as the compatibility modifier, which has a higher affinity to organic silicone oil than to hydrogenated polyisobutene at ordinary temperature. As a result, even when 25 % by mass or more of hydrogenated polyisobutene was blended, the secondary adhesion protective effect could satisfactorily be exhibited, and there was no negative effect on moldability.

Because the blending quantity of hydrogenated polyisobutene can be increased, the blending quantity of coloring material can also be increased.

In order to confirm the above, the present inventors evaluated the tranferability, for various compatibility modifiers, to the hydrogenated isobutene layer and to the organic silicone layer at ordinary temperature.

### (Migration test)

### Method

(a) Hydrogenated polyisobutene, (b) trimethyl pentaphenyl trisiloxane, and various compatibility modifiers were blended in the ratio of (a) : (b) : compatibility modifier = 3:4:1 (mass ratio), heated to 90 °C, mixed under stirring, and subsequently allowed to stand for 1 day; boundaries were compared when the mixture became 25 °C. Because of specific gravities, trimethyl pentaphenyl trisiloxane was the lower phase; thus the affinity of various compatibility modifiers or aids was estimated by the increase and decrease of the phase containing trimethyl pentaphenyl siloxane.

### Results

The results are shown in Fig. 1.

Compatibility modifiers used in the testing are as follows from the left side in Fig. 1,
1: Neopentyl glycol dicaprate
2: None (only trimethyl pentaphenyl trisiloxane)
3: Diisostearyl malate
4: Olefin oligomer
5: Glycerin triisostearate
6: Glyceryl diisostearate
7: Polyglyceryl-2 triisostearate

As observed above, neopentyl glycol dicaprate as the compatibility modifier has a higher affinity to methyl phenyl silicone than to hydrogenated polyisobutene at ordinary temperature; thus the lower layer (trimethyl pentaphenyl siloxane) increases. That is, the dilution of the hydrogenated polyisobutene layer does not take place, and good moldability can be maintained.

On the other hand, diisostearyl malate, olefin oligomer, trimethylolpropane triisostearate, etc. have a high affinity to hydrogenated polyisobutene at ordinary temperature; thus the upper layer (hydrogenated polyisobutene) increases. That is, it is considered that the dilution of the hydrogenated polyisobutene layer takes place, and moldability becomes lower.

Next, the present inventors produced samples (solid lipsticks) with the blending compositions listed in Table 1 or 2 by a conventional method. The respective samples were evaluated based on the following evaluation criteria (2).

In the blending compositions in Table 1 or 2, the mixed solution of component (b) separated when mixed with (a) at 25 °C.

### Evaluation (1): moldability

When molded into a stick-shaped lipstick, molding to the stick shape (diameter: 1.2 cm, length: 4 cm) was possible; one side was fixed, 200 g of load was applied on the other side, and the determination was based on the following evaluation criteria.

### (Evaluation criteria)

A: not broken
C: broken

### Evaluation (2): Evaluation test of long-lasting property after 2 hours

The actual usage test was conducted by 10 professional panelists. For evaluation, a sample was applied on the lip, and the long-lasting property (2 hours after application) was evaluated (scored) based on the following five-level sensory scoring criteria. The determination was by score average values and based on the following evaluation criteria.

### (Score)

5 points: very excellent
4 points: excellent
3 points: fair
2 points: poor
1 point: very poor

### (Evaluation criteria)

A+: The score average value is 4.0 point or higher and less than 5 points.
A: The score average value is 3.5 point or higher and less than 4.0 points.
B: The score average value is 2.5 point or higher and less than 3.5 points.
C: The score average value is less than 2.5 points.

### Evaluation (3): Evaluation test of the secondary adhesion protective effect

The actual usability test by 10 professional panelists was carried out. The five-level sensory evaluation (scoring) of the secondary adhesion protective effect upon application to the lip was based on the below-described scoring criteria. The determination was by the score average value based on the below-described evaluation criteria.

### (Score)

5 points: very excellent
4 points: excellent
3 points: fair
2 points: poor
1 point: very poor

### (Evaluation criteria)

A+: The score average value is 4.0 point or higher and less than 5 points.
A: The score average value is 3.5 point or higher and less than 4.0 points.
B: The score average value is 2.5 point or higher and less than 3.5 points.
C: The score average value is less than 2.5 points.

Examples marked by an asterisk (*) are not part of the claimed scope.

**[Table 1]**

| Formulation Example | | 1-1* | 1-2* | 1-3* |
|---|---|---|---|---|
| (a) | hydrogenated polyisobutene | 20 | 20 | 20 |
| (b) | trimethyl pentaphenyl trisiloxane | 53 | 53 | 53 |
| (c) | olefin oligomers | 10 | - | - |
| | glyceryl diisostearate | - | 10 | - |
| | neopentyl glycol dicaprate | - | - | 10 |
| (d) | polyethylene wax | 7 | 7 | 7 |
| (e) | coloring material, pearlescent agents | 10 | 10 | 10 |
| Evaluation (1): moldability | | A | A | A |
| Evaluation (2): Evaluation test of long-lasting property after 2 hours | | A | A | A |
| Evaluation (3): Evaluation test of the secondary adhesion protective effect | | A | A | A |

**[Table 2]**

| Formulation Example | | 2-1* | 2-2* | 2-3 |
|---|---|---|---|---|
| (a) | hydrogenated polyisobutene | 40 | 40 | 40 |
| (b) | trimethyl pentaphenyl trisiloxane | 33 | 33 | 33 |
| (c) | olefin oligomers | 10 | - | - |
| | glyceryl diisostearate | - | 10 | - |
| | neopentyl glycol dicaprate | - | - | 10 |
| (d) | polyethylene wax | 7 | 7 | 7 |
| (e) | coloring material, pearlescent agents | 10 | 10 | 10 |
| Evaluation (1): moldability | | C | C | A |
| Evaluation (2): Evaluation test of long-lasting property after 2 hours | | A | A | A+ |
| Evaluation (3): Evaluation test of the secondary adhesion protective effect | | B | B | A |

As is clear from the above Table 1, when hydrogenated polyisobutene is 20 % by mass, a good secondary adhesion protective effect and satisfactory moldability can be achieved with various compatibility modifiers or aids.

On the other hand, as shown in Table 2, when hydrogenated polyisobutene is 40 % by mass, the secondary adhesion protective effect and moldability are lowered when olefin oligomer or glyceryl diisostearate, the compatibility modifier, is used as component (c). However, when neopentyl glycol dicaprate is used, the long-lasting property can be improved while the secondary adhesion protective effect and moldability are maintained.

That is, when hydrogenated polyisobutene exceeds 20 % by mass, only neopentyl glycol dicaprate exhibits the effect as the (c) compatibility modifier.

Furthermore, the present inventors investigated the blending quantity of hydrogenated polyisobutene when neopentyl glycol dicaprate was used as the (c) compatibility modifier.

The results are shown in the following Table 3.

**[Table 3]**

| Formulation Example | | 3-1* | 3-2 | 3-3* | 3-4* |
|---|---|---|---|---|---|
| (a) | hydrogenated polyisobutene | 10 | 30 | 50 | 60 |
| (b) | trimethyl pentaphenyl trisiloxane | 63 | 43 | 23 | 13 |
| (c) | neopentyl glycol dicaprate | 10 | 10 | 10 | 10 |
| (d) | polyethylene wax | 7 | 7 | 7 | 7 |
| (e) | coloring material, pearlescent agents | 10 | 10 | 10 | 10 |
| Evaluation (1): moldability | | A | A | A | C |
| Evaluation (2): Evaluation test of long-lasting property after 2 hours | | A | A+ | A+ | A |
| Evaluation (3): Evaluation test of the secondary adhesion protective effect | | A | A | A | B |

As it is clear from the above-described results, it is understood that hydrogenated polyisobutene can be blended up to about 50 % by mass when neopentyl glycol dicaprate is used as the compatibility modifier.

**[Table 4]**

| Formulation Example | | 4-1* | 4-2 | 2-3 | 4-3 | 4-4 |
|---|---|---|---|---|---|---|
| (a) | hydrogenated polyisobutene | 40 | 40 | 40 | 40 | 40 |
| (b) | trimethyl pentaphenyl trisiloxane | 42.5 | 42 | 33 | 28 | 23 |
| (c) | neopentyl glycol dicaprate | 0.5 | 1 | 10 | 15 | 20 |
| (d) | polyethylene wax | 7 | 7 | 7 | 7 | 7 |
| (e) | coloring material, pearlescent agents | 10 | 10 | 10 | 10 | 10 |
| Evaluation (1): moldability | | A | A | A | A | A |
| Evaluation (2): Evaluation test of long-lasting property after 2 hours | | B | A | A+ | A | A |
| Evaluation (3): Evaluation test of the secondary adhesion protective effect | | C | B | A | A | B |

As it is clear from the above Table 4, a satisfactory effect can be exhibited when neopentyl glycol dicaprate is about 1 to 20% by mass.

[Table 5]

As it is clear from Table 5, when sorbitan sesquiisostearate is used as the compatibility modifier, the secondary adhesion protective effect decreases if the blending quantity of component (a) exceeds 25 % by mass. In addition, if the blending quantity of component (a) is further increased, the moldability and long-lasting property become poor.

**[Table 6]**

| Formulation Example | | 6-1* | 6-2 | 6-3 | 6-4 | 6-5* | 6-6* |
|---|---|---|---|---|---|---|---|
| (a) | hydrogenated polyisobutene | 20 | 25 | 30 | 35 | 50 | 55 |
| (b) | diphenyl dimethicone 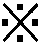 1 | 9 | 4 | - | - | - | - |
| | trimethyl pentaphenyl trisiloxane | 40 | 40 | 39 | 34 | 19 | 14 |
| | diphenylsiloxy phenyltrimethicone | 9 | 9 | 9 | 9 | 9 | 9 |
| (c) | sorbitan sesquiisostearate | - | - | - | - | - | - |
| | neopentyl glycol dicaprate | 4 | 4 | 4 | 4 | 4 | 4 |
| (d) | polyethylene wax | 7 | 7 | 7 | 7 | 7 | 7 |
| (e) | coloring material | 11 | 11 | 11 | 11 | 11 | 11 |
| Evaluation (1): moldability | | A | A | A | A | A | C |
| Evaluation (2): Evaluation test of long-lasting property after 2 hours | | A | A | A+ | A+ | A | B |
| Evaluation (3): Evaluation test of the secondary adhesion protective effect | | A | A+ | A+ | A | B | C |

As it is clear from Table 6, when neopentyl glycol dicaprate is used as component (c), the secondary adhesion protective effect can be maintained even in the case that the blending quantity of component (a) is 25% by mass or higher. In addition, when the blending quantity of component (a) is 30% by mass or higher, the long-lasting property can also be improved.

**Formulation Example 1: lipstick**

| Group of Components | Components | % by mass |
|---|---|---|
| (a) | hydrogenated polyisobutene 35% by mass | 35.0 |
| (b) | trimethyl pentaphenyl trisiloxane | 36.4 |
| (b) | diphenyl dimethicone | 1.8 |
| (c) | neopentyl glycol dicaprate | 7.0 |
| (d) | wax | 7.0 |
| (e) | coloring material | 4.6 |
| (e) | pearlescent agents | 4.6 |
| (f) | pearlescent agents | 3.0 |
| (f) | sorbitan sesquiisostearate | 2.0 |

**Formulation Example 2: lipstick**

| (a) | | |
|---|---|---|
| Group of Components | Components | % by mass |
| (a) | hydrogenated polyisobutene | 45 |
| (b) | trimethyl pentaphenyl trisiloxane | 29.9 |
| (c) | neopentyl glycol dicaprate | 10 |
| (d) | wax | 5 |
| (e) | coloring material | 4.1 |
| (e) | pearlescent agents | 1.1 |
| (f) | olefin oligomers | 2.9 |
| (f) | sorbitan sesquiisostearate | 2.0 |

**Formulation Example 3: lipstick**

| Group of Components | Components | % by mass |
|---|---|---|
| (a) | hydrogenated polyisobutene | 40 |
| (b) | trimethyl pentaphenyl trisiloxane | 35.9 |
| (c) | neopentyl glycol dicaprate | 7 |
| (d) | wax | 6 |
| (e) | coloring material | 5.1 |
| (e) | pearlescent agents | 3.1 |
| (f) | glyceryl diisostearate | 2.9 |

## Claims

1. A solid lip cosmetic, comprising:
(a) 25 to 45% by mass of hydrogenated polyisobutene;
(b) 20 to 70% by mass of one or more methyl phenyl silicones that separate at 25 °C when mixed with (a);
(c) 1 to 20% by mass of neopentyl glycol dicaprate, as the compatibility modifier, which modifies the compatibility of component (a) and component (b), and
(d) 3 to 12% by mass of wax.

2. The solid lip cosmetic according to claim 1,
wherein (e) coloring material is also contained.

3. The solid lip cosmetic according to claim 2,
wherein component (e) is dispersed in component (a).

4. The solid lip cosmetic according to any of claims 1 to 3,
wherein said methyl phenyl silicone of component (b) includes one or more selected from the group consisting of trimethyl pentaphenyl trisiloxane, diphenyl dimethicone, diphenylsiloxy phenyl trimethicone, and phenyl trimethicone.

5. The solid lip cosmetic according to any of claims 1 to 4,
wherein one or more selected from the group consisting of olefin oligomer, glyceryl tri-2-ethylhexanoate, sorbitan sesquiisostearate, propylene glycol monostearate, cetyl PEG/PPG-10/1 dimethicone, diglyceryl diisostearate, glyceryl diisostearate, pentaerythrityl tetraethylhexanoate, squalane, liquid paraffin, trimethylolpropane triisostearate, trimethylolpropane triethylhexanoate, diisostearyl malate, and cetyl ethylhexanoate are further contained as the (f) compatibility modifier aid.

## Patentansprüche

1. Festes Lippenkosmetikum, umfassend
(a) 25 bis 45 Masse-% hydriertes Polyisobuten;
(b) 20 bis 70 Masse-% eines oder mehrerer Methylphenylsilicone, die sich bei 25°C abscheiden, wenn sie mit (a) gemischt werden;
(c) 1 bis 20 Masse-% Neopentylglykoldicaprat als Verträglichkeitsmodifiziermittel, welches die Verträglichkeit der Komponente (a) und Komponente (b) modifiziert, und
(d) 3 bis 12 Masse-% Wachs.

2. Festes Lippenkosmetikum nach Anspruch 1, wobei (e) Färbematerial ebenso enthalten ist.

3. Festes Lippenkosmetikum nach Anspruch 2, wobei Komponente (e) in Komponente (a) dispergiert ist.

4. Festes Lippenkosmetikum nach irgendeinem der Ansprüche 1 bis 3, wobei das Methylphenylsilicon von Komponente (b) eines oder mehrere beinhaltet, gewählt aus der Gruppe, bestehend aus Trimethylpentaphenyltrisiloxan, Diphenyldimethicon, Diphenylsiloxyphenyltrimethicon und Phenyltrimethicon.

5. Festes Lippenkosmetikum nach irgendeinem der Ansprüche 1 bis 4, wobei eines oder mehrere, gewählt aus der Gruppe, bestehend aus Olefinoligomer, Glyceryltri-2-ethylhexanoat, Sorbitansesquiisostearat, Propylenglykolmonostearat, Cetyl-PEG/PPG-10/1-dimethicon, Diglyceryldiisostearat, Glyceryldiisostearat, Pentaerythrityltetraethylhexanoat, Squalan, flüssiges Paraffin, Trimethylolpropantriisostearat, Trimethylolpropantriethylhexanoat, Diisostearylmalat und Cetylethylhexanoat weiterhin als (f) Verträglichkeitsmodifizierhilfe enthalten sind.

## Revendications

1. Cosmétique solide pour les lèvres, comprenant :
(a) 25 à 45 % en masse de polyisobutène hydrogéné ;
(b) 20 à 70 % en masse d'un ou plusieurs méthyl phényl silicones qui se séparent à 25°C lors d'un mélange avec (a) ;
(c) 1 à 20 % en masse de dicaprate de néopentyl glycol, en tant que le modificateur de compatibilité, lequel modifie la compatibilité du composant (a) et du composant (b) ; et
(d) 3 à 12 % en masse de cire.

2. Cosmétique solide pour les lèvres selon la revendication 1, dans lequel (e) une matière colorante est également contenue.

3. Cosmétique solide pour les lèvres selon la revendication 2, dans lequel le composant (e) est dispersé dans le composant (a).

4. Cosmétique solide pour les lèvres selon l'une quelconque des revendications 1 à 3, dans lequel ledit méthyl phényl silicone du composant (b) comprend un ou plusieurs choisis dans le groupe consistant en le triméthyl pentaphényl trisiloxane, le diphényl diméthicone, le diphénylsiloxy phényl triméthicone et le phényl triméthicone.

5. Cosmétique solide pour les lèvres selon l'une quelconque des revendications 1 à 4, dans lequel un ou plusieurs choisis dans le groupe consistant en un oligomère d'oléfine, le tri-2-éthylhexanoate de glycéryle, le sesquiisostéarate de sorbitan, le monostéarate de propylène glycol, le cétyl PEG/PPG-10/1 diméthicone, le diisostéarate de diglycéryle, le diisostéarate de glycéryle, le tétraéthylhexanoate de pentaérythrityle, le squalane, la paraffine liquide, le triisostéarate de triméthylolpropane, le triéthylhexanoate de triméthylolpropane, le malate de diisostéaryle et l'éthylhexanoate de cétyle sont encore contenus en tant que l'auxiliaire modificateur de compatibilité (f).
